# EUROPEAN PATENT SPECIFICATION

(11) **EP 2 993 234 B1**
(45) Date of publication and mention of the grant of the patent: **10.10.2018**
(21) Application number: 13883590.5
(22) Date of filing: 23.08.2013
(51) Int. Cl.: C12N 15/63, C12N 15/53, C12N 5/10, A01K 67/027

(54) **CMP-ACETYLNEURAMINIC ACID HYDROXYLASE TARGETING VECTOR, TRANSGENIC ANIMAL FOR XENOTRANSPLANTATION INTRODUCED WITH THE VECTOR, AND METHOD OF MANUFACTURING THE SAME**
CMP-ACETYLNEURAMINSÄURE-HYDROXYLASE-TARGETING-VEKTOR, TRANSGENES TIER ZUR MIT DEM VEKTOR EINGEFÜHRTEN XENOTRANSPLANTATION UND VERFAHREN ZUR HERSTELLUNG DAVON
VECTEUR CIBLANT L'ACIDE CMP-ACÉTYLNEURAMINIQUE HYDROXYLASE, ANIMAL TRANSGÉNIQUE TRANSDUIT PAR LE VECTEUR POUR UNE XÉNOGREFFE, ET SON PROCÉDÉ DE PRODUCTION

(30) Priority: 30.04.2013 KR 20130047938
(43) Date of publication of application: 09.03.2016
(73) Proprietor: Konkuk University Industrial Cooperation Corp., Gwangjin-gu Seoul 05029 (KR); The Curators of the University of Missouri, Columbia, MO 65211 (US)
(72) Inventor: KIM, Jin Hoi, Seoul 05393 (KR); KWON, Deug Nam, Seoul 136-753 (KR); PARK, Jong Yi, Goseong-gun Gyeongsangnam-do 638-803 (KR); LEE, Kiho, Blacksburg, VA 24061 (US); PRATHER, Randall S., Columbia, Missouri 65201 (US); KIM, Jae Hwan, Seoul 06952 (KR); KANG, Man Jong, Gwangju 61055 (KR)
(74) Representative: Cabinet Laurent & Charras
(86) International application number: PCT/KR2013/007592
(87) International publication number: WO 2014/178485

(56) References cited:
- WO-A1-2005/033303
- WO-A2-2006/133356
- WO-A2-2009/069986
- KR-A- 20130 048 649
- US-A1- 2006 068 479
- US-A1- 2009 049 562
- US-A1- 2013 004 992
- HEDLUND MARIA ET AL: "N-glycolylneuraminic acid deficiency in mice: implications for human biology and evolution", 1 June 2007 (2007-06-01), MOLECULAR AND CELLULAR BIOLOGY, AMERICAN SOCIETY FOR MICROBIOLOGY, WASHINGTON, US, PAGE(S) 4340 - 4346, XP002547652, ISSN: 0270-7306 * page 4342, left-hand column, paragraph 1 * * page 4344, right-hand column, paragraph 2 - page 4345, left-hand column, paragraph 2 * * figure 1 *
- MBIKAY ET AL: "A targeted deletion/insertion in the mouse Pcsk1 locus is associated with homozygous embryo preimplantation lethality, mutant allele preferential transmission and heterozygous female susceptibility to dietary fat", 7 June 2007 (2007-06-07), DEVELOPMENTAL BIOLOGY, ACADEMIC PRESS, AMSTERDAM, NL, PAGE(S) 584 - 598, XP022106697, ISSN: 0012-1606 * page 585, right-hand column, paragraph 3 *
- XIAOXIA CUI ET AL: "Targeted integration in rat and mouse embryos with zinc-finger nucleases", NATURE BIOTECHNOLOGY, vol. 29, no. 1, 1 January 2011 (2011-01-01), pages 64-67, XP0055065200, ISSN: 1087-0156, DOI: 10.1038/nbt.1731
- KWON DEUG-NAM ET AL: "Production of biallelic CMP-Neu5Ac hydroxylase knock-out pigs", June 2013 (2013-06), SCIENTIFIC REPORTS, VOL. 3, PAGE(S) ARTICLE NO.: 1981, XP002755673, ISSN: 2045-2322(print) * the whole document *
- KIM, S. ET AL.: '236 Gene targeting using zinc finger nucleases (ZFN) in the porcine fibroblast' REPRODUCTION, FERTILITY AND DEVELOPMENT vol. 24, no. 1, 06 December 2011, page 230, XP008179322
- KWON, DEUG-NAM ET AL.: 'Production of biallelic CMP-Neu5Ac hydroxylase knock-out pigs' SCIENTIFIC REPORTS vol. 3, no. 1981, 13 June 2013, pages 1 - 10, XP 055256047

## Description

### [Technical Field]

The present invention relates to a CMP-acetylneuraminic acid hydroxylase targeting vector, a non-human transgenic animal for xenotransplantation introduced with the vector, and a method of manufacturing the same.

### [Background Art]

According to Korean Network for Organ Sharing (KNOS), generally, about 20,000 patients are awaiting organ transplantation in Korea each year, but the actual number of organs being donated is reportedly even less than 10% what is required for the organ transplantation. Additionally, in the U.S., the number of patients on the waiting list for organ transplantation is being added additionally one per every 16 minutes, however, 11 patients for a day on the waiting list are in a condition to face death without receiving the required organ transplantation. In this regard, the development of technologies in life science serves as a background for the development of technologies for xenotransplantation.

The continued progress in animal genetics has made it possible to produce commercially useful transgenic animals along with functional verification of each gene via removal or insertion of a particular gene. Examples of the methods for producing transgenic non-human animals include random genetic manipulation methods using microinjection or viral infection, and gene targeting methods which target particular genes using non-human embryonic stem cells or somatic cells.

The microinjection method is a conventional method to insert foreign DNA into a pronucleus of a fertilized egg and has been widely used for the manufacture of transgenic non-human animals (Harbers et al., Nature, 293(5833): 540-2, 1981; Hammer et al., Nature, 315(6021): 680-683, 1985; van Berkel et al., Nat. Biotechnol., 20(5): 484-487, 2002; Damak et al., Biotechnology (NY),14(2): 185-186, 1996). However, the efficiency of live transgenic non-human oocytes derived from the fertilized eggs, where foreign DNA was inserted via microinjection, is very low to reach only 2% to 3% (Clark et al., Transgenic Res., 9: 263-275, 2000), and it is impossible to regulate the location for a foreign gene to be inserted or to remove a particular endogenous gene therein.

The viral infection method is also widely used for the manipulation of animal genes (Soriano et al., Genes Dev., 1(4): 366-375, 1987; Hirata et al., Cloning Stem Cells, 6(1): 31-36, 2004). In the viral infection method, the gene to be inserted is introduced as a gene of an animal by a viral vector and thus this method is more efficient than the microinjection method, however, this method still is not able to insert a foreign gene into a particular location or remove a particular endogenous gene therein. Additionally, the maximum size of a gene to be inserted is limited to 7 kb, and the proteins expressed by the virus become a problem (Wei et al., Annu. Rev. Pharmacol. Toxicol., 37: 119-141, 1997; Yanez et al., Gene Ther., 5(2): 149-159, 1998).

In order to overcome the problems described above, a gene targeting technology capable of removing or inserting a particular gene may be used. The gene targeting technology was first used in the study of gene functions using mouse embryonic stem cells. Production of genetically manipulated live oocytes of a particular gene is made possible by inserting a mouse embryonic stem cell, in which the particular gene is targeted, into an embryo at the blastocyst stage by a homologous recombination. By employing the gene targeting method into a mouse embryonic stem cell, a mouse in which a large number of particular genes were targeted was produced (Brandon et al., Curr.Biol., 5(6): 625-634, 1995; Capecchi et al., Science, 244(4910): 1288-1292, 1989; Thompson et al., Cell, 56(2): 313-321, 1989; Hamanaka et al., Hum. Mol. Genet., 9(3): 353-361, 2000; Thomas et al., Cell, 51(3): 503-512, 1987; te Riele et al., Proc. Natl. Acad. Sci. USA, 89(11): 5182-5132, 1992; Mansour et al., Nature, 336(6197): 348-352, 1988; Luo et al., Oncogene, 20(3): 320-328, 2001). When the gene targeting method is applied to cattle, it is possible to produce a disease model animal which can be used in xenotransplantation using an animal bioreactor or by removing a particular gene involved in immunological rejection responses or overexpressing the gene at a particular location, and these are expected to bring a big economic benefit from the industrial aspect.

In producing gene-targeted non-human animals, the use of embryonic stem cells has been considered essential. However, the use of their stem cells in cattle has been limited although cell lines similar to the embryonic stem cells have been reported in cattle including pigs and cows (Doetschman et al., Dev. Biol., 127(1): 224-227, 1988; Stice et al., Biol. Reprod., 54(1): 100-110, 1996; Sukoyan et al., Mol. Reprod. Dev., 36(2): 148-158, 1993; Iannaccone et al., Dev. Biol., 163(1): 288-292, 1994; Pain et al., Development, 122(8): 2339-2348, 1996; Thomson et al., Proc. Natl. Acad. Sci. USA, 92(17): 7844-7848, 1995; Wheeler et al., Reprod. Fertil. Dev., 6(5): 563-568, 1994). Instead, along with the suggestion that general somatic cells as a nuclear donor cell can be used in gene targeting, the production of transgenic cattle has been made possible (Brophy et al., Nat. Biotechnol.,21(2): 157-162, 2003; Cibelli et al., Science, 280(5367): 1256-1258, 1998; Campbell et al., Nature,380(6569): 64-66, 1996; Akira Onishi et al., Science, 289;1188-1190, 2000 Denning et al., Cloning Stem Cells, 3(4): 221-231, 2001; McCreath et al., Nature, 405(6790): 1066-1069, 2000).

Meanwhile, as an optimal supplying source for xenotransplantation, miniature pigs capable of supplying a large number of organs due to similarities in the size and physiological characteristics of organs to those of humans and the fecundity are considered.

The success of porcine organ transplantation relies on whether or not a series of immunological rejection responses (hyperacute-, acute vascular-, cell-mediated-, and chronic immunological rejection responses) can be overcome. Reportedly, the hyperacute immunological rejection response, which occurs within a few minutes after transplantation, could be overcome by removing gene(s) involved in the synthesis of alpha-1,3-galactosyltransferase antigenic determinant and overexpressing human complement regulatory genes.

Specifically, a somatic cloned pig, in which alpha-1,3-galactosyltransferase (hereinafter, "GT") was heterozygously removed, was produced first by PPL Co., Ltd. of England in 2002 (Yifan Dai et al., Nat. Biotechnology, 20: 251-255, 2002). The GT gene is a gene which causes an acute immunological rejection response, and when this gene is removed it is possible to develop a disease model animal for xenotransplantation, in which the *in vivo* rejection responses are removed.

In 2003, the above company succeeded in somatic cell replication where the GT gene was removed (KR Patent Application Publication No. 10-2009-0056922) and thereby achieved a marked advance in the production of a disease model animal for organ transplantation to solve the current problem of lack of organ supply for transplantation(Carol J. Phelps, Science, 299: 411-414, 2003). In 2005, an organ, derived from a cloned pig where GT gene was removed, was transplanted into a monkey, and as a result, it was reported that the survival was sustained until 2 to 6 months after the transplantation without acute immunological rejection response (Kenji Kuwaki et al., Nature Medicine, 11(1): 29-31, 2005). However, it was reported that, even with the removal of GT gene, a serious rejection response may occur during organ transplantation by the activation of human complement genes caused by the xenoantigens which may go through with a different route (Tanemura, M. et al., Biochem. Biophys. Res. Commun., 235: 359-364, 1997; Komoda, H. et al., Xenotransplantation, 11: 237-246, 2004). In order to solve the adverse effect, there was used a method, which produces cloned pigs capable of overexpressing human complement inhibitor genes such as CD59, a decay-accelerating factor (hereinafter, "DAF"), and a membrane co-factor protein (hereinafter, "MCP"), along with the removal of GT gene (Yoichi Takahagi, Molecular Reproduction and Development 71: 331-338, 2005 Cozzi, Eb et al., Transplant Proc., 26: 1402-1403, 1994; Fodor, W. L. et al., Proc. Natl. Acad. Sci. USA 91: 11153-11157, 1994; Adams, D. H. et al., Xenotransplantation, 8: 36-40, 2001).

Meanwhile, it was reported that N-glycolylneuraminic acid (hereinafter, "Neu5Gc") antigen determinant, which is present in most mammals except humans, can also cause an immunological rejection response during xenotransplantation (WO20061133356A; Pam Tangvoranuntakul, Proc. Natl. Acad. Soc. USA 100: 12045-12050, 2003; Barbara Bighignoli, BMC genetics, 8: 27, 2007). Neu5Gc is converted from N-acetylneuraminic acid (hereinafter, "Neu5Ac") by CMAH.

Complements are protein complexes (C1-C9) consisting of proteins involved in immune responses, and they have the activities of complement fixation, in which complements, upon formation of an antigen-antibody complex, bind to cell membranes of bacterial and thereby produce holes, and an opsonization, in which complements bind to the antigen-antibody complex and promote phagocytosis. Various proteins regulating the activities of the complements have been discovered, and these regulatory proteins regulate the complements by either preventing the activation of the complements or promoting the lysis of the activated complements. The DAF presents on the cell membranes of a host cell can prevent the binding between C2 and C4b, and MCP promotes the lysis of C4b and prevents the activation of complements in a host cell thereby preventing the activation of the complements and preventing the destruction of the host cell by the complements. CD59, which is present on the surface of a host cell, can prevent the binding between C7, C8 and C5b6 and thereby prevent the formation of a membrane attack complex.

In addition to the genes which regulate the activities of complements, it was reported that thrombosis can be inhibited by the overexpression of human CD39 gene, which occurs during the xenotransplantation (US20080003212A, Karren, M. D., The Journal of Clinical Investigation, 113: 1440-1446, 2004).

According to a previous report, the expression of a foreign gene in a place other than the place it is normally expressed can cause a disorder in embryonic development, and is fatal to the nervous system which is mostly developed at the later stage of embryonic development and at the early stage after birth (Gao et al., Neurochem. Res., 24(9): 1181-1188, 1999).

KR Patent Application Publication No. 10-2009-0104328, as a relevant reference, relates to CD70 expressing neuronal stem cells and their use for prevention of immune responses in transplantation, and describes a composition for inhibiting immune responses on transplanted organs, tissues, or cells including the CD70 expressing neuronal stem cells, and a method for inhibiting immune responses of an individual using the composition.

KR Patent Application Publication No. 10-2001-0034847, as another relevant reference, relates to binding molecules derived from immunoglobulins which do not trigger complement-mediated lysis, which, as binding molecules of a recombinant polypeptide containing (i) a binding domain which can bind to a target molecule, and (ii) an effector domain including an amino acid sequence having a substantial homogeneity to the entirety or part of the constant domain of heavy chain of human immunoglobulin, are characterized in that the binding molecules can bind to target molecules without a serious complement-dependent lysis or destruction of cell-mediation of the target, and more preferably, binding molecules in which the effector domain can specifically bind to FcRn and/or FcyRIIb. In general, the binding molecules are based on the chimeric domains derived from two or more of the human immunoglobulin heavy chain CH2 domains. In an exemplary embodiment, the domain 233-236 and the domain 327-331 are corrected, and the residues beyond make the molecules null allotypic. The binding domains may be induced from arbitrary supply sources suitable for the application into the above molecules, for example, antibody, enzymes, hormones, receptors, cytokines or antigens, ligands, and adhesive molecules. Additionally, nucleic acids, host cells, production processes and materials are disclosed, for example, uses for the prevention of B cell activation, breast cell degranulation, and phagocytosis, or for the prevention of the second binding molecule to the target molecule are disclosed.

### [Disclosure]

### [Technical Problem]

The present invention is contrived by the necessities described above, and an object of the present invention is to provide a knock-out vector for a gene synthesizing xenoantigen determinant (CMAH).

Another object of the present disclosure is to provide a transgenic somatic cell line using a vector having higher efficiency and accuracy, compared to the conventional targeting vectors.

Still another object of the present disclosure is to provide a non-human cloned animal manufactured by nuclear transplantation of the non-human somatic cell line.

Still another object of the present disclosure is to provide a method for producing xeno-organs for transplantation, which are eliminated of immune rejections, including raising of non-human cloned animals followed by harvesting the organs.

### [Technical Solution]

In order to achieve the above objects, the present disclosure provides a CMP-acetylneuraminic acid hydroxylase targeting vector including CMP-acetylneuraminic acid hydroxylase 5' arm, PGKneopolyA, and CMP-acetylneuraminic acid hydroxylase 3' arm in sequential order.

In an exemplary embodiment of the present disclosure the CMP-acetylneuraminic acid hydroxylase 5' arm preferably includes the nucleotide sequence described in SEQ ID NO: 1, but it is not limited thereto.

In another exemplary embodiment of the present disclosure the PGKneopolyA preferably includes the nucleotide sequence described in SEQ ID NO: 2, but it is not limited thereto.

In still another exemplary embodiment of the present disclosure the CMP-acetylneuraminic acid hydroxylase 3' arm preferably includes the nucleotide sequence described in SEQ ID NO: 3, but it is not limited thereto.

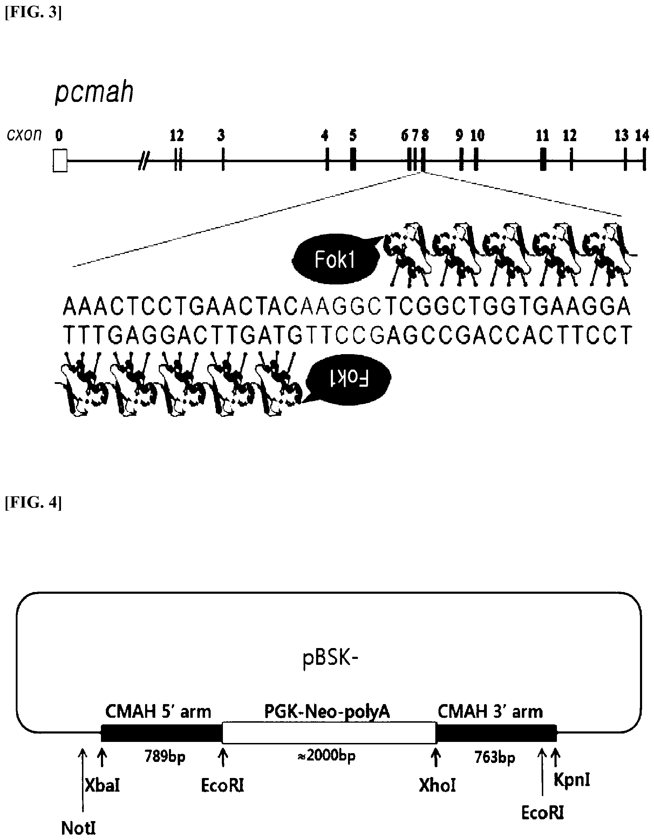

In still another exemplary embodiment of the present disclosure the CMP-acetylneuraminic acid hydroxylase targeting vector preferably includes the restriction map described in FIG. 4, but it is not limited thereto.

Additionally, the present disclosure provides a method for manufacturing knock-out cells of CMP-acetylneuraminic acid hydroxylase including transfecting the CMP-acetylneuraminic acid hydroxylase targeting vector of the present disclosure and a zinc-finger nuclease vector to cells.

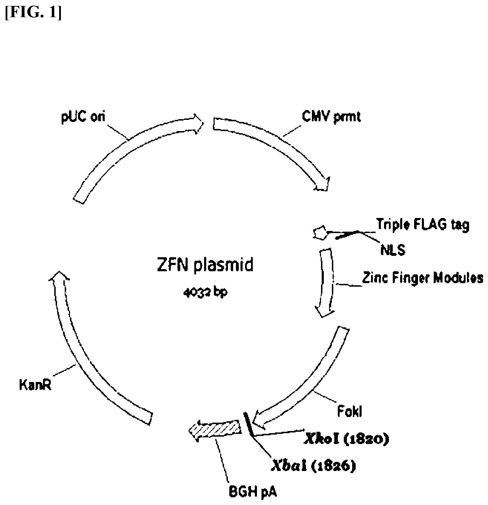

In an exemplary embodiment of the present disclosure the zinc-finger nuclease vector preferably includes the restriction map described in FIG. 1, but it is not limited thereto.

Additionally, the present disclosure provides knock-out cells of CMP-acetylneuraminic acid hydroxylase manufactured by the method of the present invention.

The above knock-out cells of the present disclosure were deposited to Division of Biological Infrastructure, Korea Research Institute of Bioscience and Biotechnology, located at Eoeun-dong, Yuseong-gu, Daejeon 34141, Korea, on July 2, 2013 under the Deposition No. of KCTC 12439BP.

Additionally, the present disclosure provides a method for manufacturing animals exclusive of humans by nuclear transplantation of the non-human knock-out cells of CMP-acetylneuraminic acid hydroxylase.

Additionally, the present invention provides a method for producing xeno-organs for transplantation, which are eliminated of immune rejections, including raising of non-human cloned animals followed by ablating the organs.

As used herein, the term "gene targeting vector" refers to a vector which can remove or insert a target gene to a particular gene location, and the vector includes a nucleotide sequence homologous to the particular gene being targeted for the occurrence of a homologous recombination.

As used herein, the term "homologous" refers to the degree of identity in nucleic acid sequence of a gene corresponding to the first domain or the second domain, and having at least 90% of identity, and preferably 95% or more of identity.

As used herein, the term "antigen determinant" refers to a region which is recognized as an antigen by an immune system of a receptor at the time of xenotransplantation, and is N-glycolylneuraminic acid (hereinafter, "Neu5Gc"), which is a cell surface glycan, and "antigen determinant synthesizing gene" refers to a gene encoding an enzyme which biosynthesizing the antigen determinant, and is CMP-acetylneuraminic acid hydroxylase (hereinafter, "CMAH") involved in the biosynthesis of Neu5Gc.

As used herein, the term "selection marker" is for the selection of transfected cells by a gene targeting vector, and markers which render selectable phenotypes such as drug resistance, nutrient requirement, resistance to cytotoxic agents, and expression of surface proteins, and refers to a marker which enables a positive selection by allowing only those cells, which express particular markers under the environment treated with a selective agent, to survive, and "selective marker gene" refers to a gene which encodes the positive selection marker, for example, neomycin phosphotransferase (hereinafter, "neo") is used for selecting stable transfected cells in eukaryotic cells, by rendering antibiotics resistance so that the eukaryotic cells can survive in a medium added with the antibiotic, neomycin.

The term "transformation" refers to an introduction of DNA into a host cell and make the DNA replicable as an extrachromosomal factor or chromosomal integration. Transformation includes any method that can introduce any given nucleic acid molecule into an organism, a cell, a tissue, or an organ, and it may be performed using any standard method suitable for a host cell as known in the art. In order to distinguish the transformation by eukaryotic cells by plasmid or nonplasmid naked DNA from the transformation by cellular tumorigenesis, it is often called "transfection", and in the present invention they are used as having the same meaning.

As used herein, the term "zinc-finger nucleases (ZFNs)" refers to artificial restriction enzymes produced by a fusion of a zinc-finger DNA binding domain to a DNA-cleaved domain. The zinc-finger domain can be manipulated for targeting DNA sequences, and this makes the zinc-finger nucleases to target the desired DNA sequences within a complex genome. It may be used to accurately change the genome of higher order species.

In the present disclosure, "DNA-cleaving domain" is used, for example, a non-specific cleaved domain derived from type II restriction enzyme FokI is typically used as a ZFNs-cleaving domain. This cleavage domain should be dimerized to cleave DNA and thus a pair of ZFNs are necessary for targeting the non-palindromic DNA domain. Standard ZFNs enable the cleaved domains to be fussed with the C-terminus of each zinc-finger domain. For the dimerization of the two cleaved domains and cleaving DNA, the two individual ZFNs should be separated at a limited distance from their C-termini and bind to the DNA on the opposite strand. It is necessary that the linker sequence most generally used between the zinc-finger domain and the cleaved domain be separated at a distance of 5 bp to 7 bp from the 5' edge in each binding domain.

A few other protein engineering technologies are adopted to improve the activities and specificities of the nuclease domains used in the ZFNs. For the production of FokI variants having improved cleaved activities, directed evolution is adopted. For the activation of only the intended hetero dimer species in order to improve the cleaved specificity of FokI by modifying the dimerized contact surfaces, a structure-based design is adopted.

The DNA-binding domain of each of the ZFNs has 3 to 6 zinc-finger repeats and can recognize the distance of from 9 bp to 18 bp. If the zinc-finger domains are completely specific to their intended target areas, even a pair 3-finger ZNFs, which can recognize an entire length of 18 bp, can theoretically target a single locus in a mammalian genome.

For enabling the binding to desired sequences, various strategies have been developed to manipulate Cys2His2 zinc-finger. They include both modular assembly and selection strategy that adopt phage display or cellular selection systems.

The most simple method to produce a new zinc-finger array is to link small zinc-finger "modules" with known specificities. The most general modular assembly process involves, for the preparation of a 3-finger array capable of recognizing 9 bp target areas, linking of three separated zinc-fingers which can recognize each of 3 bp DNA sequences. As an alternative, a method of using a 1-finger or 2-finger module to prepare zinc-finger arrays having six or more individual zinc-fingers.

Various selective methods may be used for producing zinc-finger arrays capable of targeting desired sequences. At the early stage of selection attempt, phage display was used to select proteins bound to the given DNA targets from partially randomized zinc-finger arrays from many pools. In a more recent attempt, yeast one-hybrid system, bacteria one-hybrid and two-hybrid systems, and mammalian cells were used. A more promising method to select a new zinc-finger array is to use the bacteria two-hybrid system, and is called "OPEN". This system uses the selection of a second round to obtain a 3-finger array capable of binding to a desired 9-bp sequence, after binding each zinc-finger of the pool selected in advance to be selected to bind to each given triplet. This system was developed by Zinc-Finger Consortium, as an alternative to the commercial source of manipulated zinc-finger arrays.

The present invention is described in the set of claims.

The present inventors succeeded in preparing somatic cell lines inserted with G418 (Neo) gene so that somatic cells with a knock-out CMAH gene can be efficiently selected while removing the CMAH gene involved in the synthesis of Neu5Gc antigen determinant.

The targeting vector and the cell line for transformation prepared in the present invention may be used for efficient production of cloned pigs for xenotransplantation, via complex regulation of the expression of genes involved in immunological rejection responses.

The vector used in the present disclosure is a novel technology which can produce Fox1 protein having a nuclease function of removing zinc finger proteins that can recognize the sequence of a determinant gene in a somatic cell and genes within the determinant.

The vector used in the present invention has improved efficiency and accuracy compared to that of the conventional targeting vector.

Additionally, the CMAH gene targeted by the vector is preferably the CMAH gene derived from mammals including cattle, sheep, goats, pigs, horses, rabbits, dogs, monkey, etc., more preferably the CMAH gene derived from pigs, and most preferably the CMAH gene derived from miniature pigs, but is not limited thereto.

Additionally, the vector of the present disclosure includes positive selection marker genes.

For the positive selection marker genes, neomycin phosphotransferase (Neo), hygromycin phosphotransferase (Hyg), histidinoldehydrogenase (hisD), puromycin (Puro), guanine phosphosribosyltransferase (Gpt), etc., may be used, and preferably Neo, but are not limited thereto.

When the gene targeting vector of the present disclosure is targeted into the host cell, a homologous recombination occurs between the gene for synthesizing an endogenous antigen determinant on the genome of the host cell and the targeting vector and the nucleotide sequence is substituted.

In another aspect, the present disclosure relates to a non-human transformant which is introduced with the above targeting vector.

The transformation method may include any method that can introduce any given nucleic acid molecule into a non-human organism, a cell, a tissue, or an organ, and it may be performed using any standard method suitable for a host cell as known in the art. The method may include electroporation, a calcium phosphate (CaPO₄) precipitation method, a calcium chloride (CaCl₂) precipitation method, microinjection, polyethylene glycol (PEG) method, DEAE-dextran method, a cationic liposome method, a lithium acetate-DMSO method, etc., but the method is not limited thereto.

Additionally, the present disclosure provides a non-human cloned animal to be prepared via nuclear transplantation of the non-human somatic cell line for transformation.

The non-human cloned animal may be possibly a mammal having a size similar to that of humans such as sheep, goats, pigs, dogs, etc., preferably pigs, and among them, most preferably miniature pigs.

The nuclear transplantation used for the preparation of the cloned animals may possibly be performed using a method well-known in the art, and preferably the methods described in US 6,781,030B, US 6,603,059B, US 6,235,969B, US 7,355,094B, US 7,071,372B, KR 862298B, KR 500412B, KR 807644B, JP 4153878B, US 6,700,037B, US 7,291,764B, US 6,258,998B, US 6,548,741B, WO 03/089632A, US 7,371,922B, etc., and for pigs, those described in KR500412B, KR807644, JP4153878B, US6,700,037B, KR Patent Application Publication No. 10-2009-0056922 US7,291,764B, US6,258,998B, US6,548,741B, WO03/089632A, and US 7,371,922B.

Additionally, the present disclosure provides a method for producing xeno organs for transplantation including ablating an organ necessary for transplantation, after raising the non-human cloned animals. The organ may be ablated by the conventional surgical operation after raising donor non-human cloned animals by regulating the raising period in consideration of sex, age, body weight, height, etc., of a recipient subject, and the ablated organs may be immediately transplanted to the recipient subject, or rapidly stored in a fridge.

As an optimal supplying source for xenotransplantation, miniature pigs which can supply a large-scale of organs due to the similarity in size and physiological characteristics to those of humans and their fecundity are considered. For this, miniature pigs which can control immunological rejection responses should be first produced. The success in the transplantation of pigs relies on whether or not a series of immunological rejection responses (hyperacute-, acute vascular-, cell-mediated-, and chronic immunological rejection responses) can be overcome.

According to the previous report, GT gene is a source gene causing acute immunological rejection responses during xenotransplantation, and when this gene is removed it is possible to develop a disease animal model for xenotransplantation in which biological rejection responses are removed. In 2005, an organ, derived from a cloned pig where GT gene was removed, was transplanted into a monkey, and as a result, it was reported that the survival was sustained until 2 to 6 months after the transplantation without acute immunological rejection response (Kenji Kuwaki et al., Nature Medicine, 11(1): 29-31, 2005). However, it was reported that, even with the removal of GT gene, a serious rejection response may occur during organ transplantation by the activation of human complement genes caused by the xenoantigens which may go through with a different route (Tanemura, M. et al., Biochem. Biophys. Res. Commun., 235: 359-364, 1997; Komoda, H. et al., Xenotransplantation, 11: 237-246, 2004). Meanwhile, it was reported that N-glycolylneuraminic acid (hereinafter, "Neu5Gc") antigen determinant, which is present in most mammals excluding humans, can also cause an immunological rejection response during xenotransplantation (WO20061133356A; Pam Tangvoranuntakul, Proc. Natl. Acad. Soc. USA 100: 12045-12050, 2003; Barbara Bighignoli, BMC genetics, 8: 27, 2007). Neu5Gc is converted from N-acetylneuraminic acid (hereinafter, "Neu5Ac") by CMAH. Accordingly, it is possible to regulate the acute immunological rejection response in pigs where CMAH gene is removed, and along with the pigs where GalT is removed, it is possible to develop and utilize a disease model animal for organ transplantation during xenotransplantation. According to the previous reports (Basnet NB et al., Xenotransplantation, 17: 440-448, 2010; Lutz AJ et al, Xenotransplantation, 20: 27-35, 2013), when the binding capacity to the peripheral blood mononuclear cells (PBMC) or thymocytes of a double knock-out mouse (GalT and CMAH) and a double knock-out pig (GalT and CMAH) and the natural xenoreactive antibodies within the human blood serum were examined, it was confirmed that the double knock-out mouse and pig showed reduced binding capacities compared to those of control pig and GalT knock-out. These results suggest that the acute immunological rejection response caused by xenoantigens can be controlled. According to the previous report (Kavaler et al., FASEB J, 25: 1887-1893, 2011), it was confirmed that in an obese CMAH knock-out mouse, the size of pancreas and the number of insulin-secreting cells were reduced by the pancreatic beta-cell failure.

Accordingly, in the case of the pig of the present disclosure it may be used as a model for obesity and diabetes. Additionally, according to the previous report (Chandrasekharan et al., Sci Transl Med., 28: 42-54), a CMAH knock-out mouse can be possibly used as an experimental model for Duchenne muscular dystrophy in humans. According to these reports, the pig of the present disclosure may be used as a model for muscular dystrophy.

### [Advantageous Effects of the Invention]

As can be seen in the present disclosure, a somatic cell line where G418 (Neo) gene was inserted so that somatic cells with CMAH gene knock-out can be selected while removing CMAH gene involved in the synthesis of Neu5Gc antigen determinant, and the targeting vector and the cell line for transformation prepared by the present disclosure can be used for the efficient production of cloned pigs for xenotransplantation by the complex regulation of the expression of the genes involved in the immunological rejection responses.

Additionally, to remove the CMAH protein expression in pigs, the vector of the present disclosure was so prepared that donor DNA including NEO selection factor to be inserted into the genome was prepared by homologous recombination simultaneously along with CMAH targeting vector using zinc finger nuclease (ZFN), and it was confirmed that the vector of the present disclosure has a significantly higher targeting efficiency than the conventional targeting vector, and a multiple of CMAH targeting somatic cells were selected using the ZFN and the donor DNA, and CMAH gene-targeted miniature pigs were produced by transplantation of fertilized eggs.

### [Description of Drawings]

FIG. 1 is a map of ZFN plasmid.

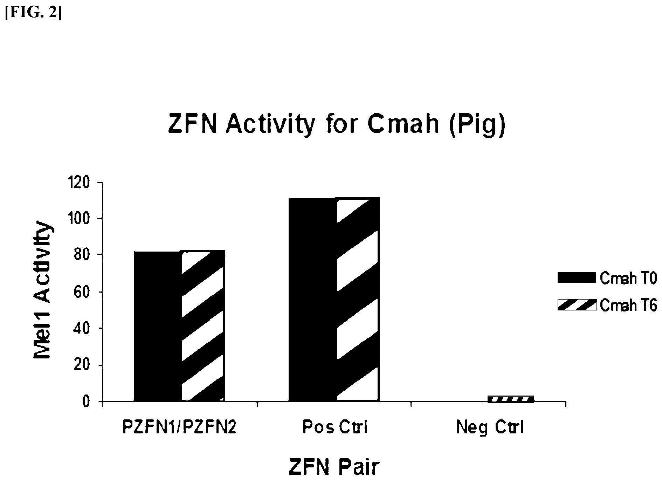

FIG. 2 is a graph of ZFN activity.
FIG. 3 is a diagram illustrating the ZFN's targeting of pig CMAH exon 8 by ZFN targeting forward & reverse plasmids.
FIG. 4 is a diagram showing a donor DNA (CMAH neo targeting vector) map.

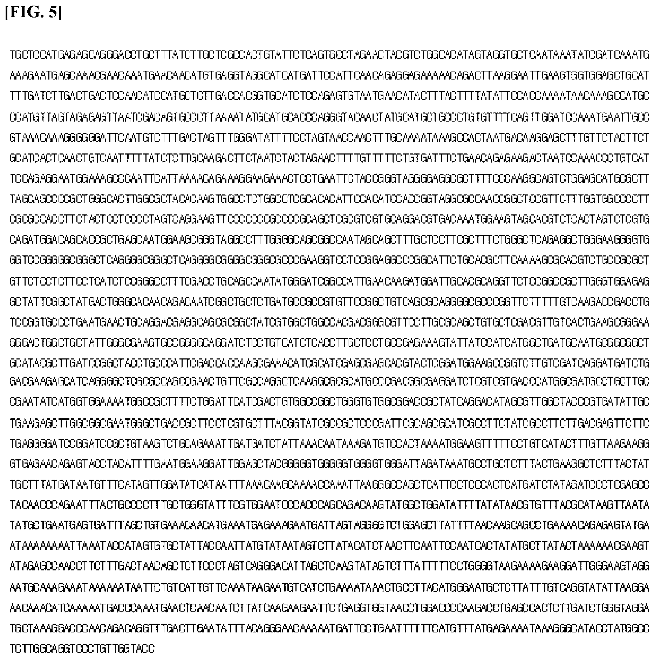

FIG. 5 is an image showing the sequence of the CMAH targeting vector, wherein yellow indicates; 5', white indicates; PGK-neo-PolyA, gray indicates; 3arm', andpBSK- sequence is not included (DNA is inserted into a XbaI-KpnI site of pBSK).

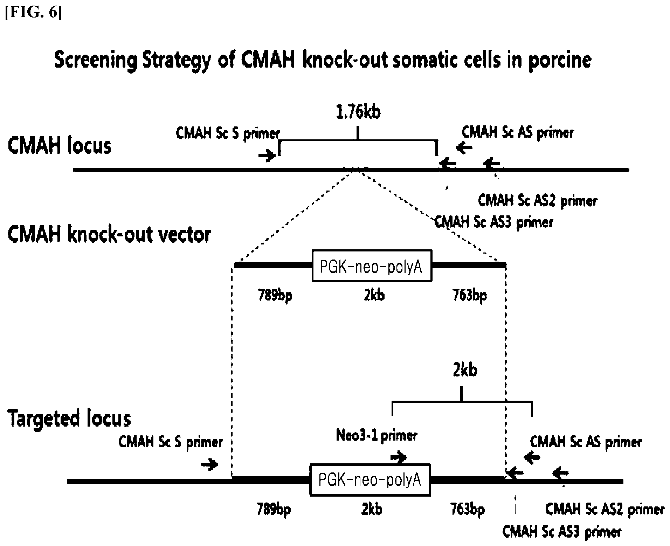

FIG. 6 is a diagram showing the screening strategy of CMAH knock-out somatic cells in pigs.

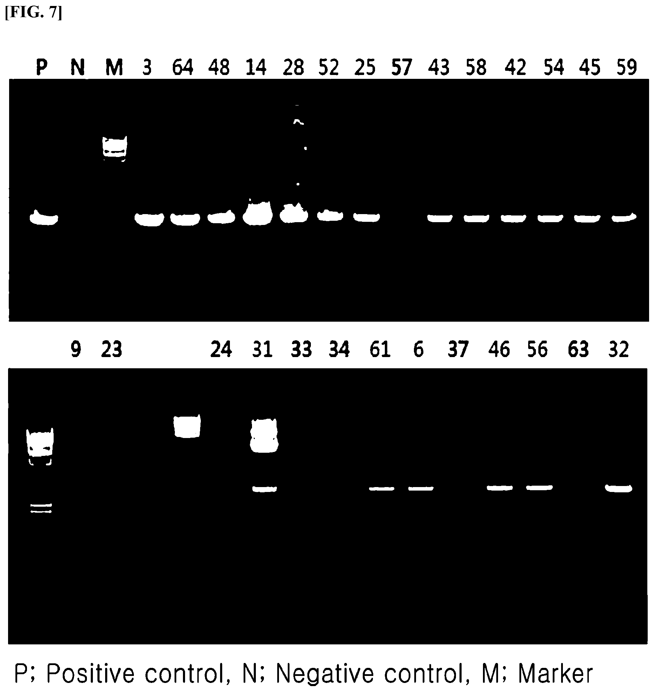

FIG. 7 is a picture showing the result of transfection by CMAH neo vector.

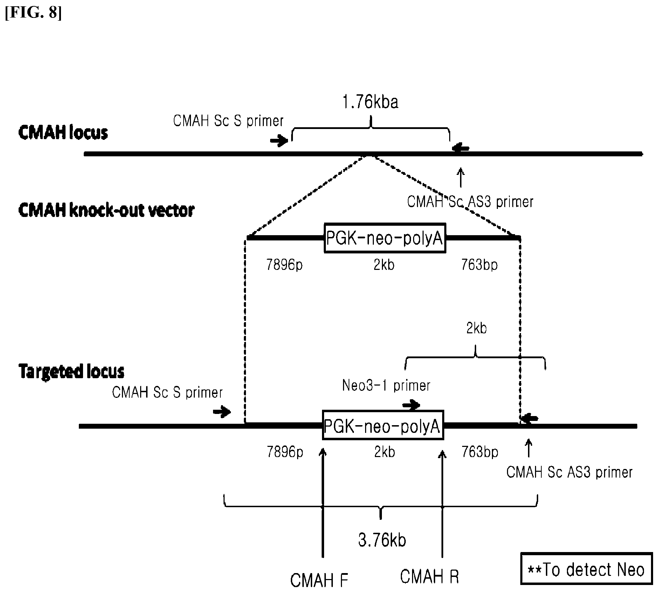

FIG. 8 is a diagram showing the locations for primer combination for confirming the transfection of CMAH knock-out pigs.

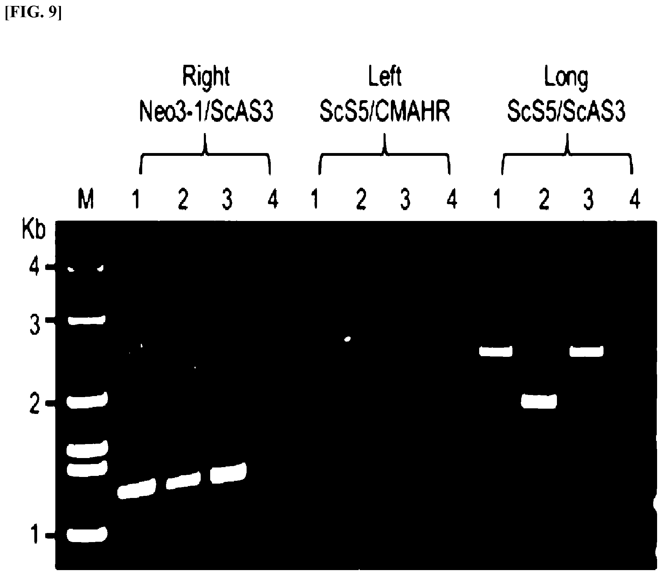

FIG. 9 is a picture showing the PCR result analyzing the presence of transfection of CMAH knock-out pigs produced by nuclear substitution.

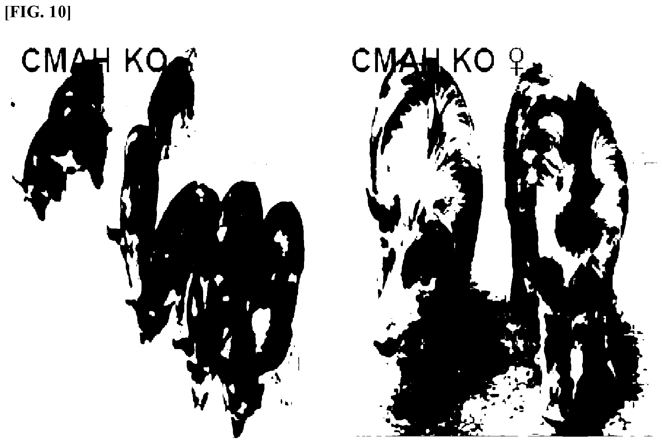

FIG. 10 shows the pictures of CMAH knock-out pigs produced by nuclear substitution.
FIG. 11 shows the results of CMAH expression in a somatic cell line established in CMAH knock-out pigs produced by nuclear substitution.
FIG. 12 shows the pictures of immunological recognition responses between a CMAH knock-out mouse model and human blood serum.
FIG. 13 shows the pictures of hearing loss according to aging in a CMAH knock-out mouse model.

### [Best Mode]

Hereinafter, the present disclosure will be described in detail with reference to non-limiting Examples. However, the exemplary embodiments disclosed herein are only for illustrative purposes and should not be construed as limiting the scope of the present invention.

### Example 1: Construction of ZFN plasmid

The construct of the present disclosure includes a T7 promoter so that it can be CMV-driven and used in *in vitro* transcription reaction. All ZFNs are triple FLAGs in N-terminus. Restriction enzymes Xho I and Xba I are used to cleave immediately after the stop codon and linearize the template for mRNA synthesis.

### Example 2: Measurement of ZFN activity

ZFN activity is measured by yeast MEL-1 reporter assay (Doyon et al., N at Biotechnol, 2008, 26(6): 702). ZFN-cleaving activity was measured before induction (0 h, blue bar) and after induction of ZFN expression (6h, red bar). MEL-1 level has a positive correlation with the ZFN activity which produces double stranded cleavage at the desired target area. After induction (6h), the ZFNs showing signals of >50% compared to the ZFN of positive control are considered as useful for genome editing experiments (even those ZFNs which show activities in an un-induced state (0h) may be excellent).

### Example 3: Preparation of donor DNA

### Construction of CMAH 5'arm-PGKneopolyA-3'arm vector

### 1) PCR cloning of 5'

First, 5'arm was obtained by PCR amplification using the genomic DNA of a Chicago miniature mini pig along with a sense primer (TCTAGACTCTCTATTTGGTGGCTCTGTTT, SEQ ID NO: 4) which includes an XbaI restriction site and an anti-sense primer (GAATTCAGGAGTTTCTTCCTTTCTGTTTT, SEQ ID NO: 5) which includes an EcoRI restriction site, and then ligated into a T-vector. The cloned DNA was confirmed to be a CMAH gene domain by DNA sequencing.

### 2) PCR cloning of 3'

3'arm was amplified by PCR using the genomic DNA of a Chicago miniature mini pig as a template along with a sense primer (CTCGAGCCTACAACCCAGAATTTACTGCC, SEQ ID NO: 6) which includes an XhoI restriction site and an anti-sense primer (GGTACCAACAGGGACCTGCCAAGAGGCCA, SEQ ID NO: 7) which includes a KpnI restriction site, and then subcloned into a T-vector. The cloned DNA was confirmed to be a CMAH gene domain by DNA sequencing.

### 3) Construction of CMAH 5'arm-PGKneopolyA-3'arm vector

The construction of CMAH 5'arm-PGKneopolyA-3'arm vector was performed by first cleaving the pKJ2 neo plasmid with EcoRI and XhoI to separate a PGKneoPolyA fragment (about 2 kb), and ligating the separated fragment into pBluscriptII (SK-) vector which was cleaved with EcoRI and XhoI, thereby obtaining pBSK-PGKneoPolyA plasmid. Regarding the 5' linking, the 5 plasmid (T-easy vector) obtained above was cleaved with XbaI and EcoRI to obtain a 789 bp fragment, and then the fragment was ligated into pBSK-PGKneoPolyA plasmid, which was cleaved with XbaI and EcoRI, thereby constructing pBSK-5'arm-PGKneoPolyA plasmid. Finally, regarding the 3' linking, the 3plasmid (T-easy vector) obtained above was cleaved with XhoI and KpnI to obtain a 763 bp fragment, and then inserted into pBSK-5'arm-PGKneoPolyA plasmid, which was cleaved with XhoI and KpnI, thereby constructing pBSK-5 'arm-PGKneoPolyA-3'arm plasmid.

### Example 4: ZFN Vector and method for transfection and selection of Donor DNA

The introduction of a gene targeting vector into a somatic cell of a miniature pig was performed via electroporation as described below. In electroporation, the cultured cells were recovered by trypsin treatment, suspended to obtain a liquid culture F10 having a cell number of 5 × 10⁶ cells/0.4 mL, and then mixed with 4.5 µg of linearized donor DNA vector and 2.6 µg each of pZFN1 and pZFN2 DNA, and the cell-vector mixture was added into a 4 mm gap cuvette. The cuvette was installed onto a BTX Electro-cell manipulator (ECM 2001), and then subjected to an electric shock under the conditions of 480V, 4 pulses, and 1 ms. After the electric shock, the cuvette was placed on ice for 10 minutes, transferred into a 10 mL liquid culture and suspended therein, and inoculated into a 48-well plate at a concentration of 1250 cells/well. 24 hours after the DNA introduction, they went through selection process for 11 days using 300 µg/mL G418. The thus-formed positive cloned somatic cells were subcultured using a 24-well culture plate and used for analysis. The thus-subcultured somatic cells were subcultured using a 12-well culture plate when they were 90% confluent in 3 to 4 days. Additionally, the cells were subcultured in a 6-well, a 60 mm culture dish, and a 100 mm culture dish at 3 to 4 day intervals, and then lyophilized or used in the experiments.

### Example 5: PCR selection of targeted cells

The PCR analysis for selection of knock-out cells was performed as described below. 100 ng of the genomic DNA separated from the cells was used as a template for PCR, and DNA was amplified using Neo3-1 primer (GCCTGCTTGCCGAATATCATGGTGGAAAAT, SEQ ID NO: 8) and CMAH Sc AS3 primer (AAGACTCCCACTTTAAAGGGTGGTGTGTAG, SEQ ID NO: 9) as primers along with Takara Ex Taq. PCR was performed under the conditions of 1 cycle at 98°C for 2 minutes; 40 cycles at 95°C for 30 seconds, 68°C for 30 seconds, 72°C for 2 minutes; and 1 cycle at 72°C for 15 minutes. After PCR, the amplified DNA was electrophoresed in a 0.8% agarose gel, and the presence/absence of about a 2 kb DNA band was finally confirmed

The results of CMAH neo vector transfection were 5 × 10⁶ cells transfection (CMAH neo vector, ZFN plasmid), by culturing in 528 of 48-well plates, having a passage of 78 single colonies, and as a result of PCR analysis 64 single colonies, 28 were shown positive at the 1^{st} PCR, and finally 19 were shown positive at the 2^{nd} PCR.

**[Table 1]**

| Number of transfected cells | Number of G418^{R} colonies | Number of G418^{R} colonies analyzed by PCR | Number of PCR -positive colonies |
|---|---|---|---|
| 5 × 10⁶ | 78 | 64 | 19 |

The materials used herein are as follows.

### Example 6: Nuclear substitution of somatic cells

For nuclear substitution, oocytes were purchased from ART (Madison, WI), matured *in vitro,* and the oocytes were enucleated and transplanted with donor cells in which CMAH knockout (KO) was targeted ([pBSK-5'arm-PGKneoPolyA-3'arm plasmid] and pZFN1 and pZFN2 DNA[CMAH knock-out] vector), and a fusion was performed by two DC pulse electric stimuli at 1.2 kV/cm. Only the survived fused oocytes were selected and transplanted into the oviduct of a surrogate mother as shown in Table 2.

**[Table 2]**

| Recipients numbers | Donor cells | No.of embryos transferred | Day of heat | Comments |
|---|---|---|---|---|
| 1 | Male ZFN C3 | 192 | 1 | 2 live |
| 2 | Male ZFN C3 | 239 | 0 | 9 live 2 stillborn |
| 3 | Female A5 | 212 | 0 | - |
| 4 | Female A9 | 221 | 0 | 1 live |
| 5 | Male D11 | 205 | 1 | - |
| 6 | Male C5 | 238 | 1 | 3 live |
| 7 | Female H10 | 246 | 1 | - |
| 8 | Male B2 | 240 | 1 | 1 with abnormality |
| 9 | Female D1 | 257 | 1 | 2 live |

Table 2 shows the results of nuclear substitution using CMAH KO cells, in which #1 and #2 indicate the ZFN transfection without donor DNA, and #3 to #9 indicate the ZFN transfection together with donor DNA.

### Example 7: Production of transgenic pigs in which CMAH knockout vector was targeted

After collecting ear tissues of offspring produced by normal delivery, the genomic DNA was extracted using GenElute™ Mammalian Genomic DNA Miniprep kit (Sigma-Aldrich). For accurate analysis based on the extracted DNA, PCR analysis was performed in combination of primers for left arm region, right arm region, and a region including both left arm and right arm, and then confirmed the presence of gene introduction.

### Combination of right arm primers

forward direction Neo 3-1: TCGTGCTTTACGGTATCGCCGCTCCCGATT, SEQ ID NO: 10
reverse direction ScAS3: AAGACTCCCACTTTAAAGGGTGGTGTGTAG, SEQ ID NO: 11

### Combination of left arm primers

forward direction ScS5: CCCTTCCATCCCACCCGTCCTCATCCTTAC, SEQ ID NO: 12
reverse direction CMAHR: ACTCTCTGTTTTCAGGCTGCTTGTT, SEQ ID NO: 13

### Combination of right arm and left arm primers

forward direction ScS5: CCCTTCCATCCCACCCGTCCTCATCCTTAC, SEQ ID NO: 14
reverse direction ScAS3: AAGACTCCCACTTTAAAGGGTGGTGTGTAG, SEQ ID NO: 15

### Example 8: Confirmation of CMAH expression in CMAH knockout pigs

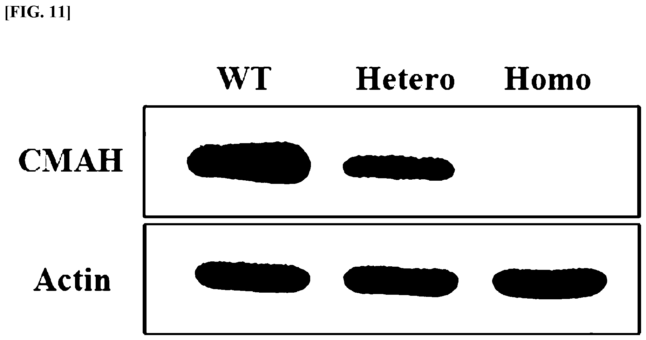

In order to confirm the presence of CMAH expression in a CMAH knockout pig, a somatic cell line was established from heterozygous and homozygous CMAH knockout pigs. Then, proteins were extracted from the cells using RIPA protein extract (Thermo Scientific, USA) solution, subjected to Western blot analysis, and thereby confirmed that CMAH protein was not expressed in the homozygous CMAH knockout pig, whereas CMAH protein expression was significantly reduced in the heterozygous CMAH knockout pig compared to that of a wild type pig. The results are shown in FIG. 11.

### Example 9: Immune recognition response between a CMAH knock-out mouse model and human serum

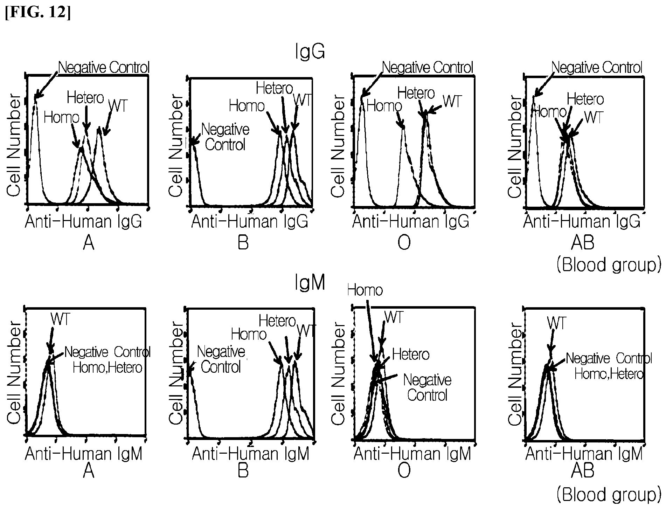

The present inventors investigated the binding capacities to the thymocytes of a CMAH knock-out mouse model and the natural xenoreactive antibodies of human sera, the binding capacity between the homozygote-derived cells and IgG in all blood types (A, B, O and AB) were reduced compared to the WT- and heterozygote-derived cells, whereas the binding capacity with IgM did not show any significance in A, O and AB blood types (FIG. 12).

### Example 10: Hearing loss due to aging in a CMAH knock-out mouse model

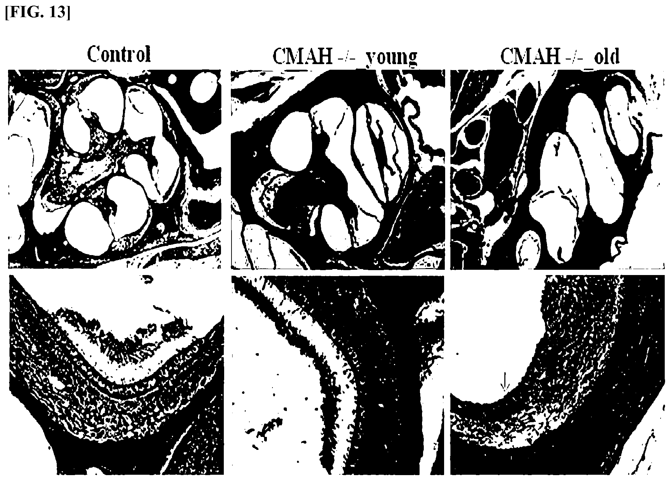

The present inventors separated cochlea from a CMAH knock-out mouse model and performed a histological analysis, and as a result, discovered abnormality in the cochlear sensory epithelium at CMAH -/- old (FIG. 13). Accordingly, the model can be used as 1) a hearing loss model according to aging and 2) a wound healing model.

### Budapest Treaty on the International Recognition of the Deposit of Microorganisms or the Purposes of Patent Procedure International Style

### Certificate

Issued pursuant to Rule 7.2 by following International deposit authorities
## Claims

1. A method for preparing a knockout cell of CMP-acetylneuraminic acid hydroxylase comprising performing a transfection of a zinc-finger nuclease vector and a CMP-acetylneuraminic acid hydroxylase targeting vector comprising CMP-acetylneuraminic acid hydroxylase 5' arm, PGKneopolyA, and CMP-acetylneuraminic acid hydroxylase 3' arm in sequential order, wherein the CMP-acetylneuraminic acid hydroxylase 5' arm consists of a nucleotide sequence described in SEQ ID NO: 1, wherein the PGKneopolyA consists of a nucleotide sequence described in SEQ ID NO: 2, and wherein the CMP-acetylneuraminic acid hydroxylase 3' arm consists of a nucleotide sequence described in SEQ ID NO: 3 into cells.

2. The method of claim 1, wherein the CMP-acetylneuraminic acid hydroxylase targeting vector has a restriction map depicted in FIG 4.

3. The method of claim 1 or 2, wherein the zinc-finger nuclease vector has a restriction map depicted in FIG. 1.

4. The knockout cells of CMP-acetylneuraminic acid hydroxylase prepared according to the method of any of claims 1 to 3.

5. The knockout cells of claim 4, which was deposited under deposition No. KCTC 12439BP.

6. A method of manufacturing animals excluding humans, comprising:
maturing obtained non-human oocytes via in vitro maturation;
enucleating the non-human oocytes; transplanting the cells of claim 4 or claim 5 excluding human cells into the enucleated non-human oocytes, and performing a fusion between them;
selecting only the survived fused oocytes, wherein said survived fused oocytes are to be transplanted into the oviduct of a non-human surrogate mother.

7. A method of producing xeno organs for transplantation eliminated of immune rejections, comprising:
raising the cloned animals, excluding humans, manufactured by the method of claim 6, wherein the organs are to be ablated from the non-human cloned animals manufactured by the method of claim 6.

## Patentansprüche

1. Ein Verfahren zur Herstellung einer aus CMP-Acetylneuraminsäure-Hydroxylase- Knockout- Zelle, umfassend die Durchführung einer Transfektion in Zellen eines Zink-Finger-Nuklease- Vektors und eines CMP-Acetylneuraminsäure-Hydroxylase-Targeting- Vektors, umfassend einen CMP-Acetylneuraminsäure-Hydroxylase 5'Arm, PGKneopolyA und einen CMP-Acetylneuraminsäure-Hydroxylase 3'Arm in sequenzieller Reihenfolge, wobei der CMP-Acetylneuraminsäure- Hydroxylase 5'Arm, aus einer Nukleotid- Sequenz besteht, beschrieben in SEQ ID NO: 1, wobei PGKneopolyA aus einer Nukleotid- Sequenz besteht, beschrieben in SEQ ID NO: 2, und wobei der CMP-Acetylneuraminsäure- Hydroxylase 3'Arm aus einer Nukleotid- Sequenz besteht, beschrieben in SEQ ID NO: 3.

2. Verfahren nach Anspruch 1, wobei der CMP- Acetylneuraminsäure-Hydroxylase- Targeting- Vektor eine Restriktionskarte hat, dargestellt in ABB. 4.

3. Verfahren nach Anspruch 1 oder 2, wobei der Zink- Finger- Nukleasevektor eine Restriktionskarte hat, dargestellt in ABB. 1

4. Die CMP-Acetylneuraminsäure- Hydroxylase- Knockout- Zellen, hergestellt entsprechend dem Verfahren in irgendeinem der Ansprüche 1 bis 3.

5. Die Knockout- Zellen, nach Anspruch 4, angemeldet mit der Anmeldungsnr. KCTC 12439BP.

6. Ein Verfahren zur Herstellung von Tieren, ausschließlich Menschen, umfassend:
Reifung gewonnener, nicht- menschlicher Oozyten durch In-vitro- Reifung;
Enukleation der nicht- menschlichen Oozyten; Transplantation der Zellen von Anspruch 4 oder Anspruch 5, ausschließlich menschlicher Zellen in die enukleierten nicht- menschlichen Oozyten und Durchführung einer Verschmelzung miteinander;
Auswahl einer der überlebenden, verschmolzenen Oozyten, wobei diese überlebenden, verschmolzenen Oozyten in den Eileiter einer nicht- menschlichen Leihmutter transplantiert werden sollen.

7. Ein Verfahren zur Herstellung von Xeno-Organen zur Transplantation ohne Immunabstoßungen, umfassend:
Aufzucht der geklonten Tiere, ausschließlich Menschen, hergestellt durch das Verfahren nach Anspruch 6, wobei die Organe von den nicht- menschlichen, geklonten Tieren, hergestellt nach dem Verfahren des Anspruchs 6 ablatiert werden sollen.

## Revendications

1. Procédé de préparation d'une cellule knock-out de l'hydroxylase de l'acide CMP-acétylneuraminique comprenant la réalisation d'une transfection dans des cellules d'un vecteur de nucléase à doigts de zinc et d'un vecteur ciblant l'hydroxylase de l'acide CMP-acétylneuraminique comprenant le bras 5' de l'hydroxylase de l'acide CMP-acétylneuraminique, PGKneopolyA, et le bras 3' de l'hydroxylase de l'acide CMP-acétylneuraminique dans un ordre séquentiel, dans lequel le bras 5' de l'hydroxylase de l'acide CMP-acétylneuraminique consiste en une séquence nucléotidique décrite à la SEQ ID NO : 1, dans lequel le PGKneopolyA consiste en une séquence nucléotidique décrite à la SEQ ID NO : 2, et dans lequel le bras 3' de l'hydroxylase de l'acide CMP-acétylneuraminique consiste en une séquence nucléotidique décrite à la SEQ ID NO : 3.

2. Procédé selon la revendication 1, selon lequel le vecteur ciblant l'hydroxylase de l'acide CMP-acétylneuraminique a une carte de restriction illustrée à la FIG.4.

3. Procédé selon la revendication 1 ou 2, selon lequel le vecteur de nucléase à doigts de zinc a une carte de restriction illustrée à la FIG.1.

4. Cellule knock-out de l'hydroxylase de l'acide CMP-acétylneuraminique préparée selon le procédé de l'une quelconque des revendications 1 à 3.

5. Cellule knock-out selon la revendication 4, laquelle a été déposée sous le No. de dépôt KCTC 12439BP.

6. Procédé de fabrication d'animaux, à l'exclusion d'humains, comprenant :
la maturation des oocytes non humains obtenus par maturation *in vitro ;*
l'énucléation des oocytes non humains ; la transplantation des cellules selon la revendication 4 ou la revendication 5, à l'exclusion des cellules humaines, dans les oocytes non humains énucléés, et la réalisation d'une fusion entre eux ;
la sélection uniquement des oocytes fusionnés survivants, selon lequel lesdits oocytes fusionnés survivants sont à transplanter dans l'oviducte d'une mère porteuse non humaine.

7. Procédé de production d'organes pour la xénotransplantation dépourvue de rejets immunitaires, comprenant :
l'élevage des animaux clonés, à l'exclusion des humains, fabriqués à l'aide du procédé de la revendication 6, selon lequel les organes sont à enlever des animaux clonés non humains fabriqués à l'aide du procédé de la revendication 6.


<110> Konkuk University Industrial Cooperation Corp.
<120> CMP-N-acetylneuraminic acid hydroxylase targeting vector, transgenic animal for xeno-organ transplantation and method of the same
<150> KR1020130047938
   <151> 2013-04-30
<160> 9
<170> KopatentIn 1.71
<210> 1
   <211> 793
   <212> DNA
   <213> Artificial Sequence
<220>
   <223> 5'arm
<400> 1
   tctagactct ctatttggtg gctctgtttt attttcttcc tagctcatca ctctttgaaa     60
   tgaacttatt tacttattca ttatttgctt ctttcactag aatgaatgct ccatgagagc    120
   agggacctgc tttatcttgc tcgccactgt attctcagtg cctagaacta cgtctggcac    180
   atagtaggtg ctcaataaat atcgatcaaa tgaaagaatg agcaaacgaa caaatgaaca    240
   acatgtgagg taggcatcat gattccattc aacagaggag aaaaacagac ttaaggaatt    300
   gaagtggtgg agctgcattt tgatcttgac tgactccaac atccatgctc ttgaccacgg    360
   tgcatctcca gagtgtaatg aacatacttt acttttatat tccaccaaaa taacaaagcc    420
   atgcccatgt tagtagagag ttaatcgaca gtgcccttaa aatatgcatg cacccagggt    480
   acaactatgc atgctgccct gtgttttcag ttggatccaa atgaattgcc gtaaacaaag    540
   gggggattca atgtctttga ctagtttggg atattttcct agtaaccaac tttgcaaaat    600
   aaagccacta atgacaagga gctttgttct acttctgcat cactcaactg tcaattttta    660
   tctcttgcaa gacttctaat ctactagaac ttttgttttt ctgtgatttc tgaacagaga    720
   agactaatcc aaaccctgtc attccagagg aatggaaagc ccaattcatt aaaacagaaa    780
   ggaagaaact cct                                                       793
<210> 2
   <211> 1642
   <212> DNA
   <213> Artificial Sequence
<220>
   <223> PGK-neo-PolyA
<400> 2
   gaattctacc gggtagggga ggcgcttttc ccaaggcagt ctggagcatg cgctttagca     60
   gccccgctgg gcacttggcg ctacacaagt ggcctctggc ctcgcacaca ttccacatcc     120
   accggtaggc gccaaccggc tccgttcttt ggtggcccct tcgcgccacc ttctactcct     180
   cccctagtca ggaagttccc ccccgccccg cagctcgcgt cgtgcaggac gtgacaaatg     240
   gaagtagcac gtctcactag tctcgtgcag atggacagca ccgctgagca atggaagcgg     300
   gtaggccttt ggggcagcgg ccaatagcag ctttgctcct tcgctttctg ggctcagagg     360
   ctgggaaggg gtgggtccgg gggcgggctc aggggcgggc tcaggggcgg ggcgggcgcc     420
   cgaaggtcct ccggaggccc ggcattctgc acgcttcaaa agcgcacgtc tgccgcgctg     480
   ttctcctctt cctcatctcc gggcctttcg acctgcagcc aatatgggat cggccattga     540
   acaagatgga ttgcacgcag gttctccggc cgcttgggtg gagaggctat tcggctatga     600
   ctgggcacaa cagacaatcg gctgctctga tgccgccgtg ttccggctgt cagcgcaggg     660
   gcgcccggtt ctttttgtca agaccgacct gtccggtgcc ctgaatgaac tgcaggacga     720
   ggcagcgcgg ctatcgtggc tggccacgac gggcgttcct tgcgcagctg tgctcgacgt     780
   tgtcactgaa gcgggaaggg actggctgct attgggcgaa gtgccggggc aggatctcct     840
   gtcatctcac cttgctcctg ccgagaaagt attatccatc atggctgatg caatgcggcg     900
   gctgcatacg cttgatccgg ctacctgccc attcgaccac caagcgaaac atcgcatcga     960
   gcgagcacgt actcggatgg aagccggtct tgtcgatcag gatgatctgg acgaagagca    1020
   tcaggggctc gcgccagccg aactgttcgc caggctcaag gcgcgcatgc ccgacggcga    1080
   ggatctcgtc gtgacccatg gcgatgcctg cttgccgaat atcatggtgg aaaatggccg    1140
   cttttctgga ttcatcgact gtggccggct gggtgtggcg gaccgctatc aggacatagc    1200
   gttggctacc cgtgatattg ctgaagagct tggcggcgaa tgggctgacc gcttcctcgt    1260
   gctttacggt atcgccgctc ccgattcgca gcgcatcgcc ttctatcgcc ttcttgacga    1320
   gttcttctga ggggatccgg atccgctgta agtctgcaga aattgatgat ctattaaaca    1380
   ataaagatgt ccactaaaat ggaagttttt cctgtcatac tttgttaaga agggtgagaa    1440
   cagagtacct acattttgaa tggaaggatt ggagctacgg gggtgggggt ggggtgggat    1500
   tagataaatg cctgctcttt actgaaggct ctttactatt gctttatgat aatgtttcat    1560
   agttggatat cataatttaa acaagcaaaa ccaaattaag ggccagctca ttcctcccac    1620
   tcatgatcta tagatccctc ga                                              1642
<210> 3
   <211> 769
   <212> DNA
   <213> Artificial Sequence
<220>
   <223> 3' arm
<400> 3
   gcctacaacc cagaatttac tgcccctttg ctgggtattt cgtggaatcc cacccagcag      60
   acaagtatgg ctggatattt tatataacgt gtttacgcat aagttaatat atgctgaatg     120
   agtgatttag ctgtgaaaca acatgaaatg agaaagaatg attagtaggg gtctggagct     180
   tattttaaca agcagcctga aaacagagag tatgaataaa aaaaattaaa taccatagtg     240
   tgctattacc aattatgtat aatagtctta tacatctaac ttcaattcca atcactatat     300
   gcttatacta aaaaacgaag tatagagcca accttctttg actaacagct cttccctagt     360
   cagggacatt agctcaagta tagtctttat ttttcctggg gtaagaaaag aaggattggg     420
   aagtaggaat gcaaagaaat aaaaaataat tctgtcattg ttcaaataag aatgtcatct     480
   gaaaataaac tgccttacat gggaatgctc ttatttgtca ggtatattaa ggaaacaaac     540
   atcaaaaatg acccaaatga actcaacaat cttatcaaga agaattctga ggtggtaacc     600
   tggaccccaa gacctgagcc actcttgatc tgggtaggat gctaaaggac ccaacagaca     660
   ggtttgactt gaatatttac agggaacaaa aatgattcct gaattttttc atgtttatga      720
   gaaaataaag ggcataccta tggcctcttg gcaggtccct gttggtacc                 769
<210> 4
   <211> 29
   <212> DNA
   <213> Artificial Sequence
<220>
   <223> primer
<400> 4
   tctagactct ctatttggtg gctctgttt 29
<210> 5
   <211> 29
   <212> DNA
   <213> Artificial Sequence
<220>
   <223> primer
<400> 5
   gaattcagga gtttcttcct ttctgtttt 29
<210> 6
   <211> 29
   <212> DNA
   <213> Artificial Sequence
<220>
   <223> primer
<400> 6
   ctcgagccta caacccagaa tttactgcc 29
<210> 7
   <211> 29
   <212> DNA
   <213> Artificial Sequence
<220>
   <223> primer
<400> 7
   ggtaccaaca gggacctgcc aagaggcca 29
<210> 8
   <211> 30
   <212> DNA
   <213> Artificial Sequence
<220>
   <223> primer
<400> 8
   gcctgcttgc cgaatatcat ggtggaaaat 30
<210> 9
   <211> 30
   <212> DNA
   <213> Artificial Sequence
<220>
   <223> primer
<400> 9
   aagactccca ctttaaaggg tggtgtgtag 30